# FASCICULE DE BREVET EUROPEEN

(11) **EP 2 303 098 B1**
(45) Date de publication et mention de la délivrance du brevet: **06.07.2016**
(21) Numéro de dépôt: 09766000.5
(22) Date de dépôt: 26.05.2009
(51) Int. Cl.: A61B 1/273, A61B 1/018, A61B 10/06

(54) **DISPOSITIF DE DÉPLACEMENT CONTROLÉ EN TRANSLATION D'UN ÉLÉMENT ALLONGÉ**
VORRICHTUNG ZUR KONTROLLIERTEN TRANSLATIONSVERSCHIEBUNG EINES LÄNGLICHEN ELEMENTS
DEVICE FOR THE CONTROLLED TRANSLATIONAL DISPLACEMENT OF AN ELONGATE ELEMENT

(30) Priorité: 26.05.2008 FR 0853422
(43) Date de publication de la demande: 06.04.2011
(73) Titulaire: Université de Strasbourg (Etablissement Public National à Caractère Scientifique, Culturel et Professionnel), 67000 Strasbourg (FR); Centre National de la Recherche Scientifique (CNRS) Etablissement Public à Caractère Scientifique et Technologique, 75016 Paris (FR)
(72) Inventeur: ZANNE, Philippe, F-67480 Roppenheim (FR); NAGEOTTE, Florent, F-68920 Wettolsheim (FR); DE MATHELIN, Michel, F-67000 Strasbourg (FR)
(74) Mandataire: Nuss, Laurent
(86) Numéro de dépôt international: PCT/FR2009/000609
(87) Numéro de publication internationale: WO 2009/153438

(56) Documents cités:
- EP-A- 1 782 744
- EP-A- 1 857 041
- WO-A-00/07500
- US-A- 3 470 876
- US-A- 4 873 965
- US-A- 5 499 632
- US-A1- 2004 133 075

## Description

La présente invention concerne de manière générale le déplacement en translation et éventuellement en rotation dans les deux sens d'un élément allongé, en particulier flexible, notamment dans un conduit ou analogue, par action au niveau d'une extrémité extérieure dudit élément, sous forme de poussée, de traction et de torsion.

Le déplacement contrôlé ou assisté de tels éléments allongés est devenu fréquent dans le domaine médical, compte tenu de l'évolution des techniques d'investigation et d'intervention.

En effet, de nouvelles approches dites mini-invasives dans différents domaines (chirurgie, gastro-entérologie, radiologie interventionnelle) consistent à amener un instrument flexible in situ par l'intermédiaire du canal opérateur d'un endoscope flexible ou via un cathéter.

L'instrument flexible est habituellement actionné manuellement de l'extérieur sous le contrôle d'images fournies par un capteur visuel à l'extrémité distale de l'endoscope ou bien également à l'aide d'un capteur per-opératoire externe (fluoroscope, échographe, ...).

Les mouvements de l'instrument flexible sont principalement des mouvements de translation dans le canal opérateur, de rotation de l'instrument autour de son axe (quand les frottements ne sont pas trop importants) et d'actionnement de l'extrémité distale de l'instrument (ouverture/fermeture pince, actionnement bistouri électrique, activation torche plasma, etc...).

La commande manuelle de l'endoscope, parfois suivant plusieurs degrés de liberté, simultanément avec la commande des mouvements de l'instrument flexible dans le canal opérateur, demande une très grande coordination oeil-main, particulièrement difficile et fatigante, même pour un praticien expérimenté.

Il existe par conséquent un besoin de libérer le praticien de certaines des manipulations précitées ou au moins de l'assister dans l'exécution de celles-ci.

Par les documents US-A-2005/0215983 et "Design of an Endoluminal NOTES Robotic System", Daniel J. Abbott et al., Proceedings of the 2007 IEEE/RSJ, San Diego, CA, USA, Oct. 29 - Nov. 2 2007, on connaît déjà des dispositifs motorisés de déplacement en translation d'instruments flexibles allongés. Dans ces documents, le déplacement en translation de l'instrument sélectionné parmi une pluralité d'instruments disponible est réalisé au moyen d'un chariot coulissant sur deux rails.

Il s'agit là d'un système mécanique encombrant, sujet à l'usure, nécessitant un espace dégagé important, monté obligatoirement sur un support spécifique et ne permettant pas une rotation de l'instrument par le praticien.

Par le document EP 1 857 041, on connaît par ailleurs un instrument médical sous la forme d'un endoscope ou analogue dont l'extrémité avant, située dans le patient, est pourvue d'un actionneur linéaire apte à déplacer en translation un élément allongé.

Toutefois, ce dispositif nécessite d'amener une alimentation électrique à l'intérieur du patient, ne permet par une manipulation manuelle de l'élément allongé, ni son échange sans extraction totale de l'endoscope.

Par le document EP 1 782 744, on connaît enfin un système endoscopique avec un instrument logé dans une gaine tubulaire.

Ce système comporte plusieurs unités d'actionnement permettant un déplacement motorisé en translation et en rotation de la gaine et un actionnement motorisé de l'instrument.

Toutefois, ces unités présentent une structure complexe et intègrent toutes des mécanismes de transformation ou de transmission du mouvement.

De plus, bien que ce système autorise un mode d'actionnement manuel, un démontage préalable de l'ensemble des unités d'actionnement motorisé doit être envisagé.

La présente invention a pour but de pallier au moins certains des inconvénients précités.

Néanmoins, l'unité de déplacement linéaire divulguée dans ce dernier document présente une constitution encombrante, lourde et complexe, du fait notamment de la mise en oeuvre d'un mécanisme de transformation d'un mouvement rotatoire en un mouvement translatif et du raccordement mécanique nécessaire avec l'élément allongé à déplacer.

De plus, cette unité de déplacement ne peut être associée, ni a fortiori intégrée, à la poignée de commande de l'endoscope et la transformation de mouvement réalisée entraîne nécessairement un jeu, préjudiciable à la précision des déplacements effectués.

La présente invention a pour but de pallier au moins certains des inconvénients précités.

Ce but est atteint grâce au dispositif de déplacement contrôlé selon la revendication 1.

A cet effet, la présente invention a pour objet un dispositif de déplacement contrôlé selon la revendication 1.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
la figure 1 est une représentation schématique partiellement par transparence d'un dispositif de déplacement contrôlé en translation d'un élément allongé selon un premier mode de réalisation de l'invention ;
la figure 2 est une vue partielle en perspective d'un système d'endoscope flexible comprenant un dispositif de déplacement tel que représenté sur la figure 1 ;
la figure 3 est une vue partielle en perspective d'une variante de réalisation du système d'endoscope représenté à la figure 2 ;
la figure 4 est une vue partielle similaire à la figure 3 d'un système d'endoscope flexible comprenant un dispositif de déplacement selon un autre mode de réalisation de l'invention, et,
les figures 5 à 7 sont des représentations schématiques partiellement par transparence de trois autres modes de réalisation de l'invention, autorisant un déplacement en translation et en rotation de l'élément allongé.

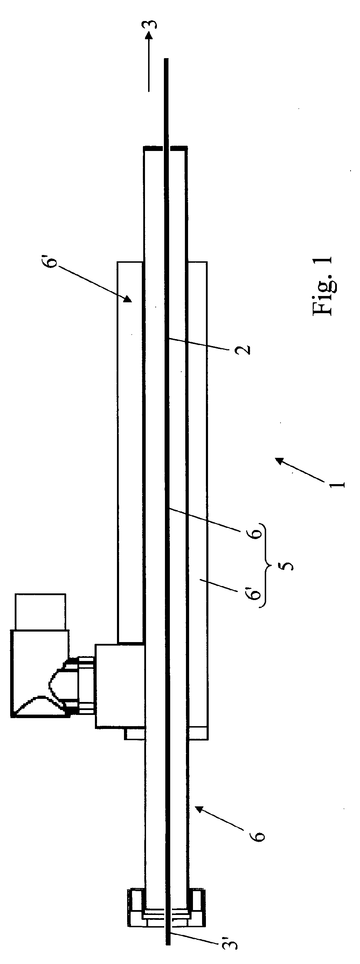

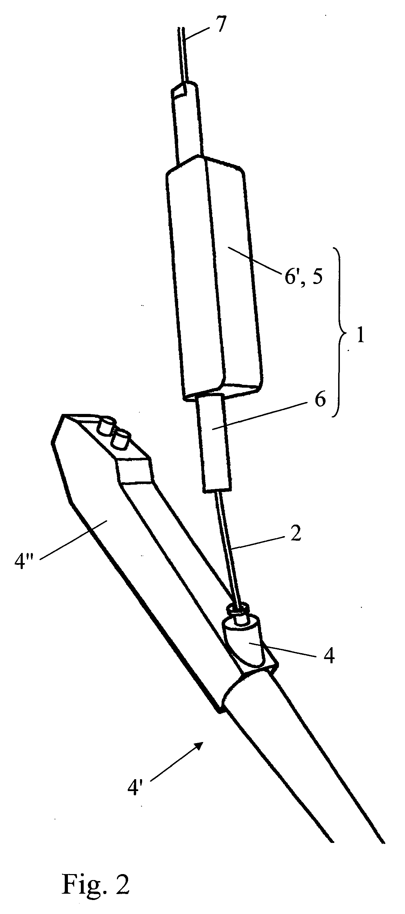

La figure 1, les figures 5 à 7 et partiellement les figures 2 à 4 montrent un dispositif 1 de déplacement contrôlé en translation et éventuellement en rotation d'un élément allongé 2 se terminant par une extrémité fonctionnelle frontale 3, notamment d'un instrument ou d'un outil destiné à être amené au niveau d'un site interne d'un patient, plus particulièrement d'un instrument d'investigation ou d'un outil de chirurgie disposé dans un cathéter ou dans un canal opérateur ou de travail 4 d'un endoscope flexible 4', ledit dispositif comprenant un actionneur linéaire 5 agissant au niveau de l'extrémité arrière 3' de l'élément allongé 2 située à l'opposée de son extrémité fonctionnelle frontale 3.

Conformément à l'invention, l'actionneur 5 consiste en un moteur linéaire à entraînement électromagnétique direct comprenant un arbre 6 mobile en translation par rapport à un stator et entraînant l'élément allongé 2 et l'extrémité arrière 3' de l'élément allongé 2 comporte une portion 7 permettant la manipulation manuelle en translation et/ou en rotation de l'élément allongé 2.

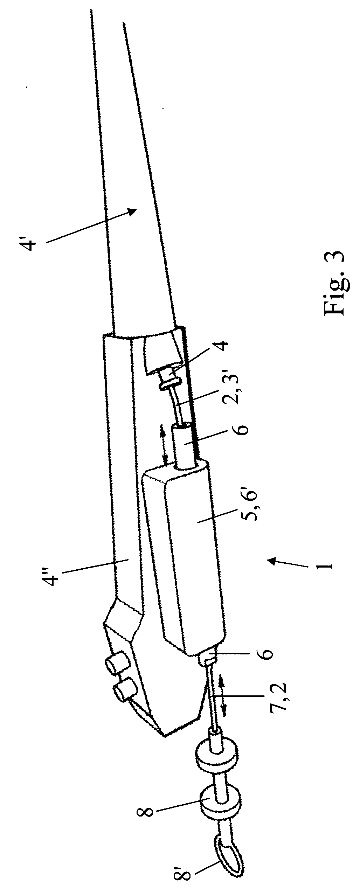

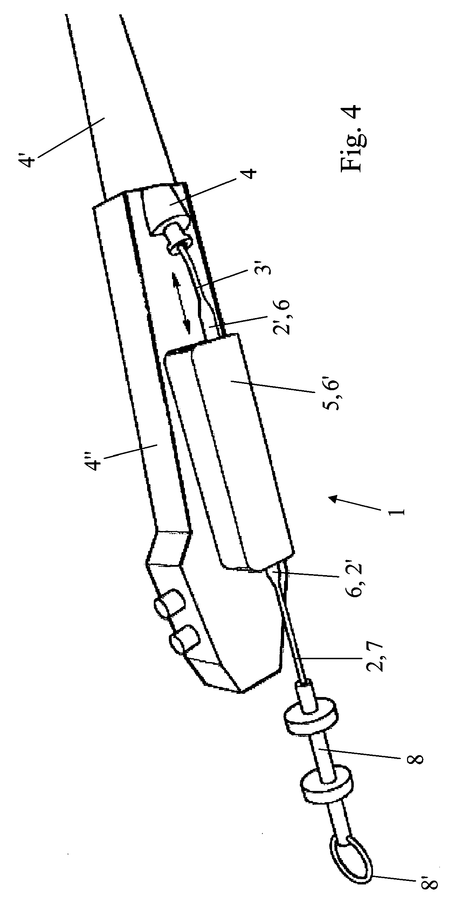

Un tel actionneur électrique linéaire 5 ne présente aucune pièce d'usure pour la transmission du mouvement, et ne nécessite aucune pièce supplémentaire pour sa solidarisation fonctionnelle avec l'élément allongé 2. De plus, il est d'une constitution simple et d'un encombrement réduit, permet une commande précise (et sans jeu) et peut être aisément associé ou monté sur un organe ou un appareil existant (Fig. 3 et 4).

L'élément allongé 2 s'étend à travers le moteur linéaire 5, ce qui facilite grandement son interchangeabilité. La solidarisation de l'élément allongé 2 avec la partie mobile (arbre 6) du moteur linéaire 5 peut être de différentes natures, tout en étant avantageusement toujours directe.

En accord avec un premier mode de réalisation de l'invention, ressortant des figures 1, 2 et 3 des dessins annexés, l'arbre mobile en translation 6 consiste en un tube creux pourvu d'aimants permanents et traversé par l'élément allongé 2, ce dernier étant solidarisé par aimantation ou par serrage avec ledit arbre mobile tubulaire 6.

Le moteur linéaire 5 peut alors, par exemple, être du type connu sous la désignation LINMOT (nom déposé) par la société NTI AG (Suisse).

La liaison par aimantation (entre les aimants de l'arbre 6 et l'élément allongé 2 au moins partiellement ferro-magnétique) présente une force suffisante pour la transmission sans glissement des mouvements de traction/poussée jusqu'à une intensité seuil, tout en permettant un dégagement aisé de l'élément allongé 2 en vue de son remplacement et un coulissement par action manuelle dudit élément 2 dans ledit arbre creux 6.

Une liaison par serrage mécanique au moyen d'une bague ou de deux bagues opposées, est également possible en relation avec la mise en oeuvre d'un arbre 6 creux, éventuellement de manière complémentaire avec la liaison magnétique, et en particulier lorsque l'élément allongé 2 est réalisé en un matériau amagnétique ou faiblement magnétique (non représenté).

Ce mode de réalisation présente ainsi plusieurs avantages parmi lesquels notamment la possibilité d'utiliser des outils et des instruments standards existants (en un matériau ferro-magnétique ou présentant au moins une partie d'extrémité arrière en un tel matériau, lorsque leur solidarisation avec l'arbre est réalisée par aimantation), la possibilité de changer manuellement de manière simple les instruments et outils 2 et la possibilité d'une manipulation conjointe ou co-manipulation de l'outil ou de l'instrument 2 avec la main et par le moteur linéaire 5.

Enfin, la solidarisation entre l'arbre 6 et l'élément allongé 2 s'effectue sur une relative grande longueur de ce dernier, et non pas de manière localisée, ce qui favorise la transmission de l'effort de poussée ou de traction.

En accord avec un deuxième mode de réalisation, représenté à la figure 4 des dessins annexés, l'arbre 6 mobile en translation est constitué par une partie 2' de l'élément allongé 2 présentant une constitution adaptée, c'est-à-dire intégrant une pluralité d'aimants permanents en série. Seule la fourniture du stator 6' est alors nécessaire.

La portion 7 d'extrémité arrière de l'élément allongé 2 dépasse du moteur linéaire 5, le cas échéant de l'arbre mobile tubulaire 6, et est pourvue d'au moins un organe 8, 8' pour la manipulation manuelle de l'élément allongé 2, éventuellement en coopération avec le moteur linéaire 5 ou en soutien ou en remplacement de ce dernier (poignée 8, anneau 8').

Afin de contrôler, le cas échéant, la profondeur et/ou la vitesse d'insertion de l'élément allongé 2, le dispositif 1 peut également comprendre un capteur de position de l'arbre mobile 6, associé ou intégré au moteur linéaire 5, permettant de mesurer le déplacement réel de l'élément allongé et sa profondeur d'enfoncement effective.

Conformément à un mode de réalisation amélioré de l'invention, et en vue de pouvoir fournir aussi un mouvement commandé en rotation de l'élément allongé 2, le dispositif 1 peut également comprendre un actionneur rotatif 9 apte à entraîner en rotation de manière contrôlée l'élément allongé 2 autour de son axe longitudinal, soit en prise directe, soit par l'intermédiaire de l'arbre mobile tubulaire 6, la portion 7 de l'extrémité arrière 3' de l'élément allongé 2 dépassant également par rapport audit actionneur rotatif 9.

Ces actionneurs translatif 5 et rotatif 9 forment ainsi une unité ou un module externe de motorisation contrôlée du déplacement de l'élément allongé 2, avec deux degrés de liberté commandée.

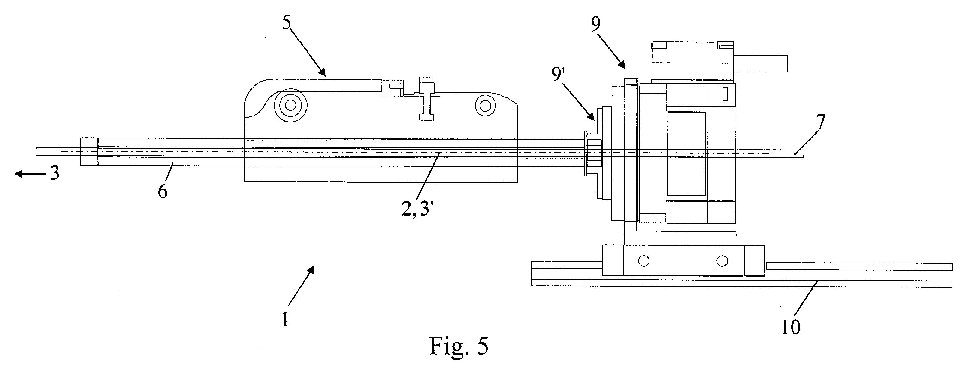

Conformément à une première variante de réalisation, représentée sur la figure 5, l'actionneur rotatif 9 peut consister en un moteur à arbre creux monté en alignement coaxial avec l'actionneur linéaire 5, solidarisé mécaniquement avec ce dernier ou avec un support 10 portant éventuellement cet actionneur linéaire 5 et couplé en rotation avec le tube creux ou la partie 2' de l'élément allongé 2 formant l'arbre mobile en translation 6, ce dernier étant libre en rotation au niveau de l'actionneur linéaire 5.

Afin de pouvoir mettre en oeuvre une version dégradée du dispositif 1, de pouvoir changer l'élément allongé 2 ou de pouvoir réaliser des manoeuvres manuelles (passage d'un fonctionnement motorisé à un fonctionnement manuel en cours d'intervention), il peut être prévu que la solidarisation mécanique et/ou le couplage en rotation du moteur à arbre creux 9 soi(en)t de nature amovible.

Le support 10 peut par exemple être pourvu d'un système de guidage linéaire de l'actionneur rotatif 9, par exemple aligné avec l'axe longitudinal de l'élément allongé 2 logé dans l'actionneur linéaire 5. L'actionneur rotatif 9 est avantageusement pourvu d'une pièce 9' de raccord ou d'accouplement (mécanique ou magnétique) permettant de solidariser en rotation le tube 6 ou l'élément allongé 2 avec ledit actionneur 9, préférentiellement de manière amovible.

Le moteur à arbre creux 9 peut par exemple se présenter sous la forme d'un moteur du type connu sous la désignation "Servo Actuator" de la société Harmonic Drive Systems Inc.

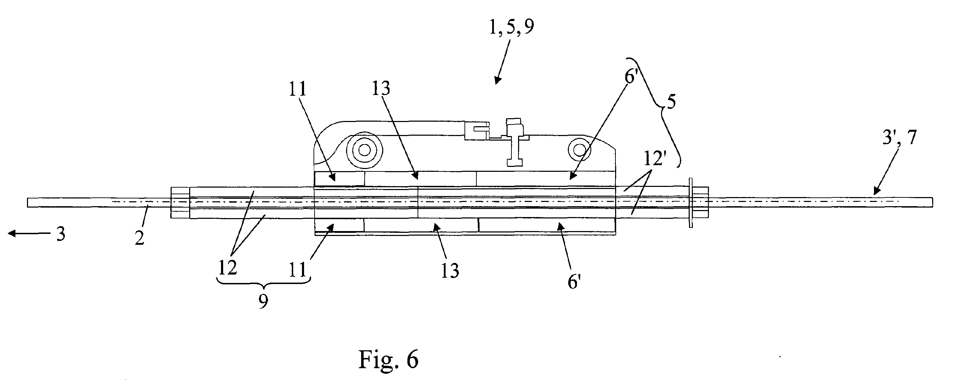

Selon une seconde variante de réalisation, et comme le montre la figure 6 des dessins annexés, l'actionneur rotatif 9 consiste en un moteur magnétique intégré dans le moteur linéaire 5, l'arbre 6 formant ainsi un rotor pouvant être entraîné en translation et en rotation.

L'arbre 6 sous forme de tube peut alors être formé de deux parties, à savoir une première partie 12 destinée à former le rotor de l'actionneur rotatif 9 et une seconde partie 12' destinée à former le coulisseau (ou rotor coulissant) de l'actionneur linéaire 5. Le dispositif de déplacement comporte alors, au niveau de son passage traversant recevant l'arbre 6, un premier stator 6' destiné à interagir avec la seconde partie 12' en vue du déplacement en translation dudit arbre 6 et un second stator 11 destiné à interagir avec la première partie 12 en vue du déplacement en rotation de cet arbre 6. Les deux stators étant séparés axialement par une zone magnétiquement inactive, assurant éventuellement une fonction de guidage.

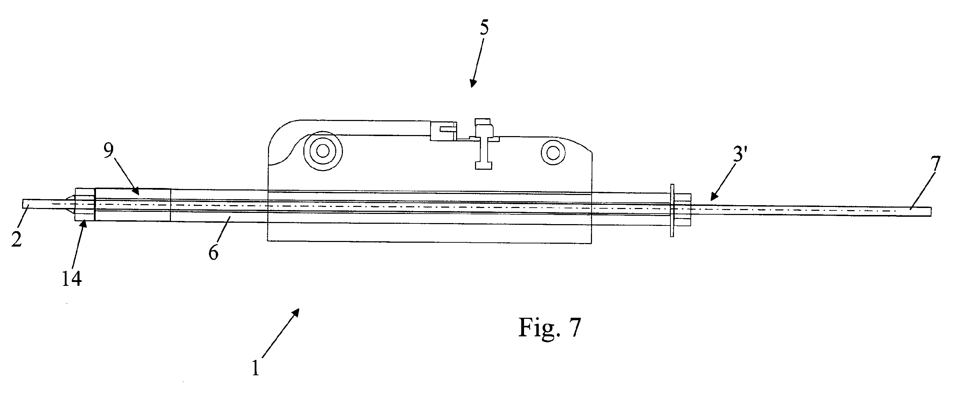

Enfin, en accord avec une troisième variante de réalisation, ressortant de la figure 7, l'actionneur rotatif 9 consiste en un moteur axial intégré dans l'arbre creux 6 recevant l'élément allongé 2, ce moteur axial pouvant consister en un moteur à arbre creux de faible diamètre (apte à être intégré dans l'arbre 6).

La solidarisation en rotation entre le moteur 9 et l'élément allongé 2 est obtenue par l'intermédiaire d'un moyen de serrage, par exemple de type mandrin, permettant d'accoupler le moteur 9 à l'élément 2.

Avantageusement, le guidage en translation de l'arbre creux 6 intégrant le moteur axial à arbre creux 9 (par exemple une version miniaturisée du moteur 9 de la première variante précitée) s'effectue avec blocage en rotation dans l'actionneur linéaire 5.

Enfin, il peut aussi être prévu que le dispositif 1 comprenne ou soit associé à une unité de commande, automatique ou manuelle assistée, contrôlant le déplacement de l'arbre mobile 6 par l'intermédiaire d'un asservissement en effort ou en vitesse de l'actionneur et/ou rotatif et/ou par l'intermédiaire d'un asservissement en position. La commande du dispositif de déplacement 1 peut être automatique dans le cadre d'un système robotisé d'investigation ou d'intervention ou être du type à commande manuelle assistée, par exemple sous la forme d'un organe de commande électrique.

La présente invention a également pour objet, comme le montrent partiellement les figures 2 à 4, un système d'investigation et/ou de chirurgie, en particulier système d'endoscope flexible 4', comprenant au moins un instrument ou outil 2 sous forme d'élément allongé flexible.

Ce système est caractérisé en ce qu'il comprend également au moins un dispositif 1 de déplacement contrôlé en translation et éventuellement en rotation tel que décrit ci-dessus, associé audit au moins un instrument ou outil 2.

Le dispositif de déplacement 1 peut constituer un module ou une unité séparé(e), mais il peut également être prévu que le moteur linéaire 5 soit monté sur la ou une poignée de commande 4" du système 4', ainsi qu'éventuellement l'actionneur rotatif 9. Dans ce dernier cas, le moteur linéaire 5 est fixé de manière à ne pas interférer avec les boutons de commande de la poignée 4".

Le système d'endoscope flexible 4' peut par exemple consister en un système tel que décrit et représenté dans la demande de brevet PCT n° PCT/FR2008/050312 du 25 février 2008.

Enfin, le système peut en outre comprendre au moins un actionneur supplémentaire apte à commander l'outil ou l'instrument intégré à l'élément allongé 2, par exemple par l'intermédiaire de câbles, associé à l'actionneur linéaire et/ou rotatif 5, 9.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Dispositif de déplacement contrôlé en translation, et éventuellement en rotation, d'un élément allongé se terminant par une extrémité fonctionnelle frontale, notamment d'un instrument ou d'un outil destiné à être amené au niveau d'un site interne d'un patient, plus particulièrement d'un instrument d'investigation ou d'un outil de chirurgie disposé dans un cathéter ou dans un canal opérateur ou de travail d'un endoscope flexible, ledit dispositif comprenant un actionneur linéaire agissant au niveau de l'extrémité arrière de l'élément allongé située à l'opposée de son extrémité fonctionnelle frontale et à l'extérieur du patient, ledit actionneur (5) consistant en un moteur linéaire à entraînement électromagnétique direct comprenant un arbre (6) mobile en translation par rapport à un stator et entraînant l'élément allongé (2), **caractérisé en ce que** ledit élément allongé traverse ledit moteur linéaire (5),
**en ce que** ledit arbre mobile (6) consiste soit en un tube creux pourvu d'aimants permanents et traversé par l'élément allongé (2), avec une solidarisation par aimantation ou serrage avec ledit arbre (6), soit en une partie (2') de l'élément allongé (2) intégrant une pluralité d'aimants permanents en série, et
**en ce que** l'extrémité arrière (3') de l'élément allongé (2) comporte une portion (7) dépassant du moteur linéaire (5), le cas échéant de l'arbre mobile tubulaire (6), et pourvue d'au moins un organe (8, 8') permettant la manipulation manuelle en translation et/ou en rotation de l'élément allongé (2), en coopération avec le moteur linéaire (5) ou en soutien ou en remplacement de ce dernier.

2. Dispositif selon la revendication 1, , **caractérisé en ce qu'**il comprend également un capteur de position de l'arbre mobile (6), associé ou intégré au moteur linéaire (5).

3. Dispositif selon l'une quelconque des revendications 1 et 2, **caractérisé en ce qu'**il comprend également un actionneur rotatif (9) apte à entraîner en rotation de manière contrôlée l'élément allongé (2) autour de son axe longitudinal, soit en prise directe, soit par l'intermédiaire de l'arbre mobile tubulaire (6), la portion (7) de l'extrémité arrière (3') de l'élément allongé (2) dépassant également par rapport audit actionneur rotatif (9).

4. Dispositif selon la revendication 3, **caractérisé en ce que** l'actionneur rotatif (9) consiste en un moteur à arbre creux monté en alignement coaxial avec l'actionneur linéaire (5), solidarisé mécaniquement avec ce dernier ou avec un support (10) portant éventuellement cet actionneur linéaire (5) et couplé en rotation avec le tube creux ou la partie (2') de l'élément allongé (2) formant l'arbre mobile en translation (6), ce dernier étant libre en rotation au niveau de l'actionneur linéaire (5).

5. Dispositif selon la revendication 4, **caractérisé en ce que** la solidarisation mécanique et/ou le couplage en rotation du moteur à arbre creux (9) sont de nature amovible.

6. Dispositif selon la revendication 3, **caractérisé en ce que** l'actionneur rotatif (9) consiste en un moteur magnétique intégré dans le moteur linéaire (5), l'arbre (6) formant ainsi un rotor pouvant être entraîné en translation et en rotation.

7. Dispositif selon la revendication 3, **caractérisé en ce que** l'actionneur rotatif (9) consiste en un moteur axial intégré dans l'arbre creux (6) recevant l'élément allongé (2).

8. Dispositif selon l'une quelconque des revendications 1 à 7, **caractérisé en ce qu'**il comprend en outre une unité de commande (4', 4"), automatique ou manuelle assistée, contrôlant le déplacement de l'arbre mobile (6) par l'intermédiaire d'un asservissement en effort ou en vitesse de l'actionneur linéaire et/ou rotatif et/ou d'un asservissement en position.

9. Système d'investigation et/ou de chirurgie, en particulier système d'endoscope flexible, comprenant au moins un instrument ou outil sous forme d'élément allongé flexible, **caractérisé en ce qu'**il comprend également au moins un dispositif (1) de déplacement contrôlé en translation et éventuellement en rotation selon l'une quelconque des revendications 1 à 8, associé audit au moins un instrument ou outil (2).

10. Système selon la revendication 9, **caractérisé en ce que** le moteur linéaire (5) est monté sur la ou une poignée de commande (4") du système (4'), ainsi qu'éventuellement l'actionneur rotatif (9).

11. Système selon la revendication 9 ou 10, **caractérisé en ce qu'**il comprend en outre au moins un actionneur supplémentaire apte à commander l'outil ou l'instrument intégré à l'élément allongé (2), par exemple par l'intermédiaire de câbles, associé à l'actionneur linéaire et/ou rotatif (5, 9).

## Patentansprüche

1. Vorrichtung zur kontrollierten Verschiebungs- und gegebenenfalls Drehbewegung eines länglichen Teils, das vorne in einem Arbeitsende endet, insbesondere eines Instrumentes oder Werkzeuges, das dazu bestimmt ist, in den Bereich einer Stelle im Inneren eines Patienten gebracht zu werden, insbesondere eines Untersuchungsinstrumentes oder eines chirurgischen Instrumentes, das sich in einem Katheter oder dem Arbeits- oder Instrumentenkanal eines flexiblen Endoskops befindet, wobei die genannte Vorrichtung ein lineares Betätigungsorgan umfasst, das im Bereich des hinteren Endes des länglichen Teiles, das sich am gegenüber dem vorderen Arbeitsende entgegengesetzten Ende und außerhalb des Patienten befindet, angreift wobei das genannte Betätigungsorgan (5) aus einem Linearmotor mit elektromagnetischem Direktantrieb besteht, eine gegenüber einem Stator verschiebungsbewegliche Spindel (6) umfasst und das längliche Teil (2) mitführt, **dadurch gekennzeichnet, dass** das genannte längliche Teil den genannten Linearmotor (5) durchquert,
dadurch, dass die genannte bewegliche Spindel (6) entweder aus einem hohlen Rohr besteht, das mit Permanentmagneten versehen ist und vom länglichen Teil (2) durchquert wird, unter Befestigung an der genannten Spindel (6) durch Magnetisierung oder Klemmung, oder aus einem Teil (2') des länglichen Teils (2), das eine Reihe von Permanentmagneten enthält, und
dadurch, dass das hintere Ende (3') des länglichen Teils (2) einen Abschnitt (7) aufweist, der aus dem Linearmotor (5) herausragt, gegebenenfalls aus der rohrförmigen beweglichen Spindel (6), und mit mindestens einem Organ (8, 8') versehen ist, das die manuelle Betätigung zur Verschiebung oder Drehung des länglichen Teils (2) erlaubt, unter Zusammenwirkung mit dem Linearmotor (5) oder zu dessen Unterstützung oder stattdessen.

2. Vorrichtung nach Patentanspruch 1, **dadurch gekennzeichnet, dass** sie ebenfalls einen Sensor der Stellung der beweglichen Spindel (6) umfasst, mit dem Linearmotor (5) verbunden oder in ihn integriert.

3. Vorrichtung nach irgendeinem der Patentansprüche 1 und 2, **dadurch gekennzeichnet, dass** sie ebenfalls ein Drehbetätigungsorgan (9) umfasst, geeignet, in kontrollierter Weise das längliche Teil (2) um seine Längsachse zu drehen, entweder direkt oder über die rohrförmige, bewegliche Spindel (6), wobei der Abschnitt (7) des hinteren Endes (3') des länglichen Teils (2) ebenfalls relativ zum genannten Drehbetätigungsorgan (9) herausragt.

4. Vorrichtung nach Patentanspruch 3, **dadurch gekennzeichnet, dass** das Drehbetätigungsorgan (9) aus einem Motor mit Hohlwelle besteht, der koaxial mit dem Linearbetätigungsorgan (5) ausgerichtet montiert ist, mit diesem mechanisch fest verbunden oder mit einem Träger (10), der gegebenenfalls dieses Linearbetätigungsorgan (5) trägt und zur gemeinsamen Drehung mit dem hohlen Rohr oder dem Teil (2') des länglichen Teils (2), der die verschiebungsbewegliche Spindel (6) bildet, gekoppelt ist, wobei diese letztgenannte in Höhe des linearen Betätigungsorgans (5) frei drehbar ist.

5. Vorrichtung nach Patentanspruch 4, **dadurch gekennzeichnet, dass** die mechanische Verbindung und/oder die Drehkopplung des Hohlwellenmotors (9) lösbar sind.

6. Vorrichtung nach Patentanspruch 3, **dadurch gekennzeichnet, dass** das Drehbetätigungsorgan (9) aus einem Magnetmotor besteht, der in den Linearmotor (5) integriert ist, so dass die Spindel (6) einen Rotor bildet, der zu Translations- und Rotationsbewegungen veranlasst werden kann.

7. Vorrichtung nach Patentanspruch 3, **dadurch gekennzeichnet, dass** das Drehbetätigungsorgan (9) aus einem Axialmotor besteht, der in die Hohlwelle (6) integriert ist, die das längliche Teil (2) aufnimmt.

8. Vorrichtung nach irgendeinem der Patentansprüche 1 bis 7, **dadurch gekennzeichnet, dass** sie außerdem eine Steuereinheit (4', 4"), automatisch oder manuell assistiert, umfasst, die die Verschiebung der beweglichen Spindel (6) über eine Kraft- oder Geschwindigkeitsregelung des linearen und/oder Drehbetätigungsorgans und/oder eine Stellungsregelung steuert.

9. Untersuchungs- und/oder chirurgisches System, insbesondere System mit flexiblem Endoskop, mindestens ein Instrument oder Werkzeug in Form eines flexiblen, länglichen Teils umfassend, **dadurch gekennzeichnet, dass** es auch mindestens eine Vorrichtung (1) zur kontrollierten Verschiebungs- und gegebenenfalls Drehbewegung nach irgendeinem der Patentansprüche 1 bis 8 in Verbindung mit dem genannten mindestens einen Instrument oder Werkzeug (2) umfasst.

10. System nach Patentanspruch 9, **dadurch gekennzeichnet, dass** der Linearmotor (5) auf dem oder einem Betätigungsgriff (4") des Systems (4') montiert ist, wie auch gegebenenfalls das Drehbetätigungsorgan (9).

11. System nach Patentanspruch 9 oder 10, **dadurch gekennzeichnet, dass** es außerdem mindestens ein weiteres Betätigungsorgan umfasst, das in der Lage ist, das Werkzeug oder das Instrument beispielsweise über Seile oder Drahtseile zu betätigen und das in das längliche Teil (2) integriert ist, das mit dem linearen und/oder Drehbetätigungsorgan (5, 9) verbunden ist.

## Claims

1. Device for controlled translational displacement, and optionally rotational displacement, of an elongated element that terminates in a frontal functional end, in particular an instrument or a tool that is designed to be brought to an internal site in a patient, more particularly an exploratory instrument or a surgical tool placed in a catheter or in an operating channel or working channel of a flexible endoscope, whereby said device comprises a linear actuator that acts at the rear end of the elongated element that is located opposite its frontal functional end and outside of the patient,
said actuator (5) consisting of a direct-drive electromagnetic linear motor that comprises a shaft (6) that moves in translation relative to a stator and that drives the elongated element (2), **characterized**
**in that** said elongated element passes through said linear motor (5),
**in that** said shaft that moves in translation (6) consists either of a hollow tube that is provided with permanent magnets and through which the elongated element (2) passes, whereby the latter is made integral by magnetization or by tightening with said tubular movable shaft (6), or of a portion (2') of the elongated element (2) incorporating a plurality of permanent magnets in series, and
**in that** the rear end (3') of the elongated element (2) comprises a portion (7) extending beyond the linear motor (5), possibly beyond the tubular moving shaft (6), and provided with at least one organ (8, 8') that makes possible the manual manipulation in translation and/or in rotation of the elongated element (2), in cooperation with the linear motor (5) or in support or in replacement of the latter.

2. Device according to claims 1, **characterized in that** it also comprises a position sensor of the movable shaft (6), combined with or integrated in the linear motor (5).

3. Device according to any of claims 1 and 2, **characterized in that** it also comprises a rotary actuator (9) that can drive in rotation, in a controlled manner, the elongated element (2) around its longitudinal axis, either directly or by means of the tubular movable shaft (6), whereby the portion (7) of the rear end (3') of the elongated element (2) also extends relative to said rotary actuator (9).

4. Device according to claim 3, **characterized in that** the rotary actuator (9) consists of a motor with a hollow shaft that is mounted in coaxial alignment with the linear actuator (5), mechanically made integral with the latter or with a support (10) that optionally bears this linear actuator (5) and coupled in rotation with the hollow tube or the portion (2') of the elongated element (2) that forms the movable shaft in translation (6), whereby the latter is free in rotation at the linear actuator (5).

5. Device according to claim 4, **characterized in that** the mechanical integration and/or the coupling in rotation of the motor with hollow shaft (9) are detachable in nature.

6. Device according to claim 3, **characterized in that** the rotary actuator (9) consists of a magnetic motor that is integrated in the linear motor (5), the shaft (6) thus forming a rotor that can be driven in translation and in rotation.

7. Device according to claim 3, **characterized in that** the rotary actuator (9) consists of an axial motor that is integrated in the hollow shaft (6) that accommodates the elongated element (2).

8. Device according to any of claims 1 to 7, **characterized in that** it also comprises an automatic or assisted manual control unit (4', 4"), controlling the displacement of the movable shaft (6) by means for controlling the force or speed of the linear and/or rotary actuator and/or by means for controlling the position.

9. Exploratory and/or surgical system, in particular a flexible endoscope system, comprising at least one instrument or tool in the form of a flexible elongated element, **characterized in that** it also comprises at least one device (1) for controlled translational displacement and optionally rotational displacement according to any of claims 1 to 8, combined with said at least one instrument or tool (2).

10. System according to claim 9, **characterized in that** the linear motor (5) is mounted on the or a control handle (4") of the system (4') as well as optionally the rotary actuator (9).

11. System according to claim 9 or 10, **characterized in that** it also comprises at least one additional actuator that can control the tool or the integrated instrument with the elongated element (2), for example by means of cables, combined with the linear and/or rotary actuator (5, 9).
